# EUROPEAN PATENT APPLICATION

(11) **EP 4 722 726 A1**
(43) Date of publication of application: **08.04.2026**
(21) Application number: 24815002.1
(22) Date of filing: 15.04.2024
(51) Int. Cl.: G01N 35/00, G01N 27/26, G01N 27/333, G01N 27/416

(54) **AUTOMATIC ANALYZER AND MEASURING METHOD USING THE SAME**

(30) Priority: 30.05.2023 JP 2023088599
(71) Applicant: HITACHI HIGH-TECH CORPORATION, Tokyo 105-6409 (JP)
(72) Inventor: NISHIZAKI, Hitomi, Tokyo 105-6409 (JP); USHIKU, Emiko, Tokyo 105-6409 (JP); FURUYA, Miki, Tokyo 105-6409 (JP)
(74) Representative: Strehl & Partner mbB
(86) International application number: PCT/JP2024/015047
(87) International publication number: WO 2024/247515

(57) **Abstract**

In the prior art, the reliability of a measurement result is lowered by the deterioration of an ISE reagent remaining in a flow passage in the standby time of an electrolyte analysis unit. To solve this problem, when receiving an instruction to measure a patient sample requiring measurement using an electrolyte analysis unit 8, this automatic analysis device 1 determines deterioration of a residual reagent remaining in the flow path of the electrolyte analysis unit prior to measurement of the patient sample when the standby time of the electrolyte analysis unit is equal to or greater than a predetermined time.

## Description

### Technical Field

The present invention relates to an automatic analyzer that performs qualitative/quantitative analysis of components of blood, urine, or the like collected from a patient sample and a measuring method using the same.

### Background Art

Automatic analyzers are used in a hospital laboratory and a testing center. Sample testing performed in the hospital laboratory needs to be started promptly in the event of an emergency even during surgery, at night, or on holidays, and a highly reliable measurement result needs to be obtained therefrom in the same manner as from regular testing. The automatic analyzer has a colorimetric analysis unit and an electrolyte analysis unit, and the electrolyte analysis unit using an ion selective electrode method uses three types of reagents (hereinafter referred to as the ISE reagents), i.e., an internal standard solution, a diluent, and a reference electrode solution. Accordingly, when the ISE reagents have deteriorated, a correct measurement result cannot be obtained. In general, the deterioration of the reagents is difficult to sense, since it is a gradual, day-to-day change with only slight variations. Patent Literature 1 discloses sensing of measurement value abnormalities resulting from the reagent deterioration from a fluctuation pattern in daily calibration results (for about one month).

### Citation List

### Patent Literature

Patent Literature 1: Japanese Unexamined Patent Application Publication No. 2013-213841

### Summary of Invention

### Technical Problem

In Patent Literature 1, the abnormalities can be sensed only at the timing of calibration. In addition, the abnormalities are sensed from the fluctuation pattern, and deterioration of the ISE reagents (referred to as the residual reagents) remaining in flow paths, which occurs during a standby time of the analyzer, is not assumed. An object of the present invention is to sense deterioration of residual reagents, which occurs during a standby time of an analyzer, and increase reliability of a result of measurement by the automatic analyzer. Solution to Problem

An automatic analyzer according to an embodiment of the present invention, includes: an electrolyte analysis unit that measures an electrolyte concentration by an ion selective electrode method and can measure at least a chloride ion concentration; and a control unit. On receipt of an instruction to measure a patient sample that requires measurement using the electrolyte analysis unit, the control unit determines, when a standby time of the electrolyte analysis unit is equal to or longer than a predetermined time, deterioration of a residual reagent remaining in a flow path of the electrolyte analysis unit prior to the measurement of the patient sample.

### Advantageous Effects of Invention

When the standby time of the electrolyte analysis unit is equal to or longer than the predetermined time, the deterioration of the residual reagents is determined before the measurement of the patient sample to eliminate measurement value abnormalities resulting from the deterioration of the ISE reagents in the flow path and improve reliability of a measurement result. Other problems and novel features will be apparent from the description of the present specification and accompanying drawings.

### Brief Description of Drawings

FIG. 1 is a hardware configuration diagram of an automatic analyzer.
FIG. 2 is a schematic configuration diagram of an electrolyte analysis unit.
FIG. 3 is a diagram illustrating a result of continuously measuring an accuracy control sample with a standby time of 0 hours.
FIG. 4 is a diagram illustrating a result of continuously measuring the accuracy control sample with a standby time of 2 hours.
FIG. 5 is a diagram illustrating a result of continuously measuring the accuracy control sample with a standby time of 5 hours.
FIG. 6 is a diagram illustrating a result of continuously measuring the accuracy control sample with a standby time of 17 hours.
FIG. 7 is a flow chart of sample measurement by the electrolyte analysis unit.
FIG. 8 is a flow chart of determination of ISE reagent deterioration.
FIG. 9 is a diagram for illustrating a method of determining a predetermined time X serving as an implementation reference for the determination of the ISE reagent deterioration.
FIG. 10 illustrates details of a flow chart of the determination of the ISE reagent deterioration in a first embodiment.
FIG. 11 illustrates an example of a screen for the determination of the ISE reagent deterioration in the first embodiment.
FIG. 12 illustrates details of a flow chart of the determination of the ISE reagent deterioration in a second embodiment.
FIG. 13 illustrates an example of the screen for the determination of the ISE reagent deterioration in the second embodiment.

### Description of Embodiments

Embodiments of the present invention will be described below with reference to the drawings. It goes without saying that, in the following embodiments, the components (including elements, steps, and the like) are not necessarily indispensable unless specifically indicated or unless the components are considered to be obviously indispensable in principle.

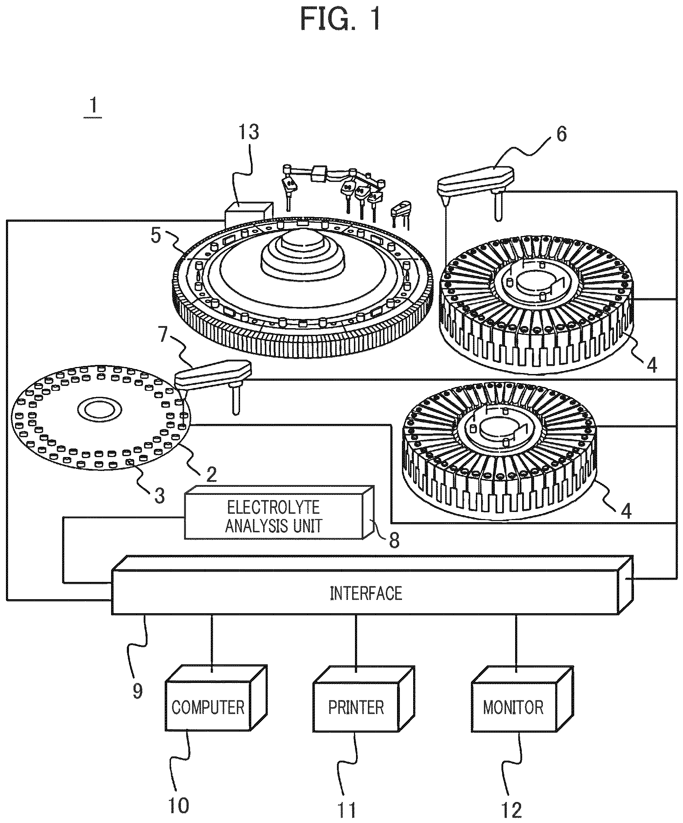

Referring to FIG. 1, a description will be given of an overall configuration of an automatic analyzer in the present embodiment. FIG. 1 is a hardware configuration diagram illustrating the overall configuration of the automatic analyzer.

An automatic analyzer 1 is a sample testing device that automatically analyzes a sample collected from a patient. The automatic analyzer 1 uses a sample dispensing mechanism 7 and a reagent dispensing mechanism 6 to dispense given amounts of the patient sample and a reagent and cause a reaction therebetween. A description is given herein by taking a disk-type automatic analyzer as an example, but the present invention is not limited thereto, and is also applicable to, e.g., a rack type.

The automatic analyzer 1 includes a colorimetric analysis unit and an electrolyte analysis unit. The colorimetric analysis unit includes, as major components thereof, a sample disk 2 that is mounted with a sample cup 3 for containing a sample and rotates a disk-shaped table to move any sample to a sampling position, reagent disks 4 that are mounted with reagent bottles for containing reaction reagents and rotated to move any of the reagents to a dispensing position, a reaction container 5 that is held at a given temperature by constant temperature water to cause a reaction between the given amounts of individually dispensed patient sample and reagents, the reagent dispensing mechanism 6 that dispenses the given amount of the reagent from any of the reagent bottles into the reaction container 5, the sample dispensing mechanism 7 that dispenses the given amount of the sample from the sample cup 3 into the reaction container 5, a photometer 13 that measures progress of the chemical reaction between the sample and the reagents by absorption photometry, and the like. An electrolyte analysis unit 8 measures an electrolyte concentration by an ion selective electrode method using electrodes that selectively react to specific ions.

A computer (control unit) 10 is connected to each of the mechanisms of the automatic analyzer 1 via an interface 9. The computer 10 controls each of the mechanisms, while a measurement result obtained by each of the analysis units is input to the computer 10. The computer 10 calculates a sample concentration from the measurement result and outputs the obtained sample concentration to a printer 11 and to a monitor 12.

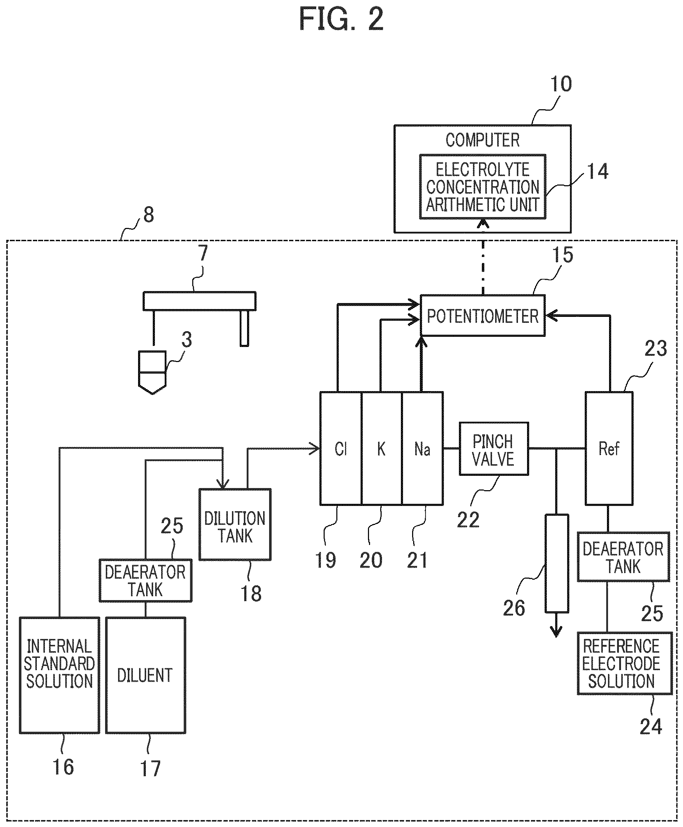

FIG. 2 is a configuration diagram of the electrolyte analysis unit 8 using the ion selective electrode method. Referring to FIG. 2, a description will be given of an overall configuration of the electrolyte analysis unit 8. The electrolyte analysis unit 8 includes ion selective electrodes including sensitive membranes for target ions, which are specifically an Na (sodium) ion electrode 21, a K (potassium) ion electrode 20, and a Cl (chloride) ion electrode 19, a reference electrode 23, and a potentiometer 15 that measures potential differences between the individual ion selective electrodes 19 to 21 and the reference electrode 23. Aspiration of the sample or the reagents to the ion selective electrodes or to the reference electrode is performed by a sipper syringe 26. By switching of a flow path by a pinch valve 22, either the aspiration to the ion selective electrode or the aspiration to the reference electrode 23 is performed.

A basic operation in the ion selective electrode method is performed using measurement of an internal standard solution 16, measurement of the sample, and the measurement of the internal standard solution 16 as one set, and respective concentrations of Na ions, K ions, and Cl ions can be measured at a time by one measurement.

In the measurement of the internal standard solution 16, after the internal standard solution 16 is ejected into a dilution tank 18, the internal standard solution 16 is aspirated to the ion selective electrodes 19 to 21, while a reference electrode solution 24 is aspirated to the reference electrode 23, and a potential difference between each of the ion selective electrodes 19 to 21 and the reference electrode 23 is measured with the potentiometer 15. Thus, for the internal standard solution 16, an electromotive force of each of the ion selective electrodes 19 to 21 subjected to electromotive force correction is measured. Note that the reference electrode solution 24 is subjected to bubble removal by a deaerator tank 25 and aspirated to the reference electrode 23.

In measurement of the sample, a given amount of the sample is dispensed by the sample dispensing mechanism 7 from the sample cup 3 into the dilution tank 18, while a given amount of a diluent 17 is ejected into the dilution tank 18 to dilute the sample to a given factor. Note that the diluent 17 is subjected to the bubble removal by the deaerator tank 25 and ejected into the dilution tank 18. Then, the diluted sample is aspirated to the ion selective electrodes 19 to 21, while the reference electrode solution 24 is aspirated to the reference electrode 23, and the potential difference between each of the ion selective electrodes 19 to 21 and the reference electrode 23 is measured with the potentiometer 15. Thus, for the diluted sample, the electromotive force of each of the ion selective electrodes 19 to 21 subjected to the electromotive force correction is measured.

On the basis of a difference between the electromotive force of each of the ion selective electrodes 19 to 21 measured for the internal standard solution 16 and the electromotive force of each of the ion selective electrodes 19 to 21 measured for the diluted sample, a concentration of the diluted sample can be calculated. The calculation of the concentration is performed in an electrolyte concentration arithmetic unit 14 of the computer 10. Note that the electrolyte concentration arithmetic unit 14 can be implemented as software by an electrolyte concentration arithmetic program that arithmetically determines an electrolyte concentration from a potential difference and can be implemented by the computer 10.

Thus, in the ion selective electrode method, for the calculation of the concentration, the ISE reagents (internal standard solution 16, the diluent 17, and the reference electrode solution 24) are used. In addition, in terms of a structure of the electrolyte analysis unit 8, the internal standard solution 16 and the diluent 17 are ejected into the dilution tank 18 through flow paths, while the reference electrode solution 24 is aspired to the reference electrode 23 through a flow path, and therefore the electrolyte analysis unit 8 is maintained in a state where the flow paths are constantly filled with the respective ISE reagents.

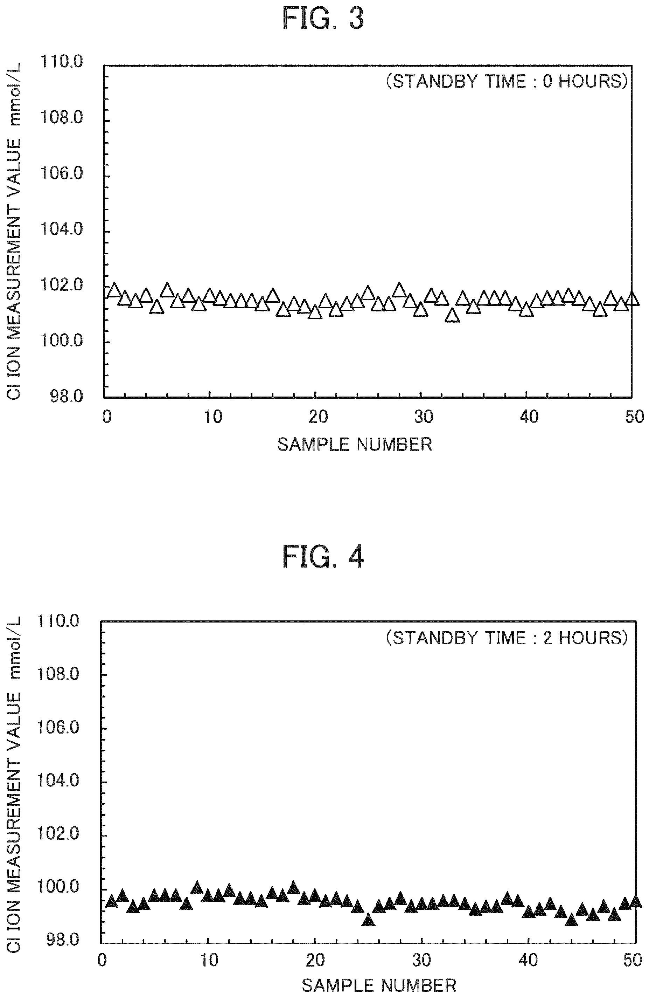

Referring to FIGS. 3 to 6, a description will be given of deterioration of the ISE reagents in the flow paths. FIGS. 3 to 6 show a result of continuously measuring Cl ions for the 50 accuracy management samples after the electrolyte analysis unit 8 was left on standby for a predetermined time. The respective standby times in FIGS. 3 to 6 are 0, 2, 5, and 17 hours. Each of the standby times mentioned herein refers to a time from when the reagents are primed in the electrolyte analysis unit 8 until the continuous measurement is started. The priming of the reagents refers to processing of supplying the ISE reagents to the respective flow paths from the individual ISE reagent bottles and replacing the ISE reagents remaining in the flow paths with the ISE reagents from the individual ISE reagent bottles.

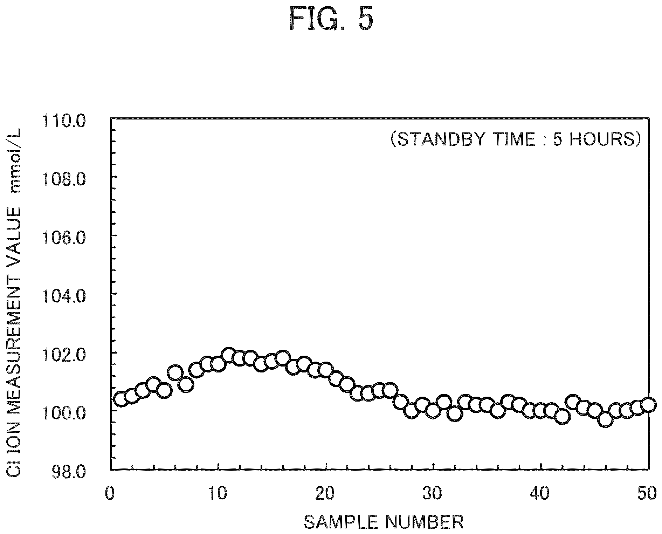

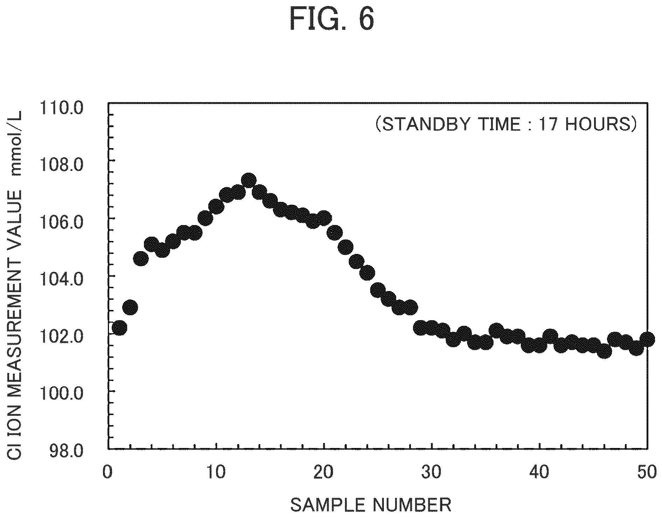

As a result of a comparison between these measurement results, an increase was observed in a variation of a Cl ion measurement value when the standby time was extended. Specifically, in a first half of the continuous measurement, as the standby time was longer, mountain-shaped fluctuations of the measurement value are more noticeable while, in a second half of the continuous measurement, each of the fluctuations of the measurement value was smaller. It can be considered that a factor causing such fluctuations is responding of the Cl ion electrode to a component resulting from the deterioration of the ISE reagents remaining in the flow paths. The Cl ion electrode 19 responds not only to the Cl ions, but also to other ions (referred to as interfering ions) having properties similar to those of the Cl ions. Accordingly, when the deterioration of the ISE reagents causes the interfering ions, a measurement value of the Cl ions consequently increases. Even though the deterioration of the ISE reagents equally proceeds in the bottles and in the flow paths, the interfering ions caused in relatively small amounts of the ISE reagents staying in the flow paths are at relatively high concentrations. A conceivable reason for the gradually increasing Cl ion measurement value in FIG. 5 and FIG. 6 is the presence of a concentration gradient of a component resulting from the deterioration of the residual reagents in the flow paths. The replacement of the ISE reagents deteriorated in the flow paths with the ISE reagents from the bottles stabilizes the Cl ion measurement value. Timing with which the measurement value gradually increases and a peak appears depends on a volume of each of the flow paths, and therefore differs from one analyzer model to another. Such fluctuations of the ion measurement value are not observed with the Na ions and the K ions.

Therefore, in the present embodiment, when the standby time is equal to or longer than a predetermined time, before the patient sample is measured, the electrolyte analysis unit measures the flow path confirmation sample (first embodiment) or the colorimetric analysis unit measures the component resulting from the deterioration of the ISE reagents (second embodiment) to thereby determine the presence or absence of the deterioration of the residual reagents, selectively determine a maintenance type from a determination result on an analyzer side, and notify the user of the determined maintenance type.

### First Embodiment

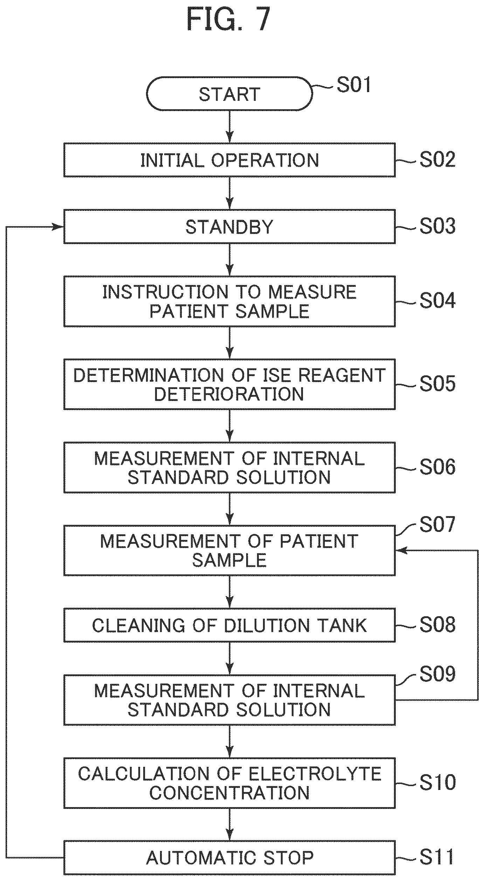

Referring to FIG. 7, a description will be given of a sample-side measuring operation of the electrolyte analysis unit 8. Each of steps is controlled by the computer 10.

S01: User activates the automatic analyzer 1. By inputting of an activation instruction by the user to the computer 10, each of the mechanisms including the electrolyte analysis unit 8 is activated via the interface 9.

S02: An initial operation is started. Details of an initial operation are set in advance by the user. In a case of the electrolyte analysis unit 8, the initial operation mostly include reagent priming typically including cleaning of each of the mechanisms included in the electrolyte analysis unit 8 and replacement of the ISE reagents in the flow paths with new reagents from the bottles.

S03 to S04: Standby is continued until a patient sample measurement instruction including electrolyte analysis as a measurement item is given. By receiving the patient sample measurement instruction (S04) from the computer 10, a standby status (S03) is ended, and the sample measuring operation moves to Step S05.

S05: In the present embodiment, the deterioration of the ISE reagents is determined before electrolyte analysis is started on receipt of the patient sample measurement instruction (S04). Details thereof will be described later. In Steps S06 to S09 shown below, the electrolyte analysis unit 8 analyzes an electrolyte of the patient sample, which is specifically as follows.

S06: While the pinch valve 22 is kept closed, the reference electrode solution 24 is aspired with the sipper syringe 26 to the reference electrode 23. At this time, the reference electrode solution 24 is degassed by flowing through the deaerator tank 25. Meanwhile, in a state where the internal standard solution 16 is ejected into the dilution tank 18 and the pinch valve 22 is open, the internal standard solution 16 in the dilution tank 18 is aspired with the sipper syringe 26 to the Cl ion electrode 19, to the K ion electrode 20, and to the Na ion electrode 21 in the dilution tank 18. Note that the residual solution remaining in the dilution tank 18 is aspired with a vacuum nozzle (not shown) to be discarded. Subsequently, a potential difference (electromotive force) between the internal standard solution 16 and the reference electrode solution 24 is measured with the potentiometer 15.

S07: After the patient sample was dispensed and the diluent 17 was ejected into the dilution tank 18, agitation is performed to mix the patient sample and the diluent 17. At this time, the diluent 17 flows through the deaerator tank 25 to be degassed. Subsequently, in the same manner as in Step S06, the diluted sample was measured. In other words, while the pinch valve 22 is kept closed, the reference electrode solution 24 is aspired with the sipper syringe 26 to the reference electrode 23. Meanwhile, while the pinch valve 22 is kept open, the diluted sample in the dilution tank 18 is aspired with the sipper syringe 26 to the Cl ion electrode 19, to the K ion electrode 20, and to the Na ion electrode 21. Subsequently, a potential difference (electromotive force) between the diluted sample and the reference electrode solution 24 is measured with the potentiometer 15. Note that the residual solution remaining in the dilution tank 18 is aspired with the vacuum nozzle to be discarded.

Note that, in the present flow, a description has been given of the measurement of the patient sample, but a standard solution to be used for calibration and the accuracy control sample to be used for measurement accuracy control are also measured by the same method as described herein.

S08: The dilution tank 18 is cleaned.

S09: The internal standard solution 16 is measured in the same manner as in S06. When the measurement instruction is continued, the sample measurement operation returns to Step S07 where the patient sample is measured. By thus performing the continuous measurement by looping Steps S07 to S09, the measurement can be ended with the measurement of the internal standard solution, and consequently a transition can be made to a standby status with the flow paths from, e.g., the dilution tank 18 to the ion selective electrodes being filled with the internal standard solution 16.

S10: The potential difference measured with the potentiometer 15 is input to the computer 10 via the interface 9, and the computer 10 calculates the electrolyte concentration of the patient sample on the basis of a result of measuring the internal standard solution and a result of measuring the patient sample. The obtained concentration is output to the printer 11 or to the monitor 12. When the continuous measurement is performed, the processing in Step S10 can be performed in parallel with the measurement by the electrolyte analysis unit 8.

S11: The electrolyte analysis unit 8 automatically stops, and transitions to the standby status (S03).

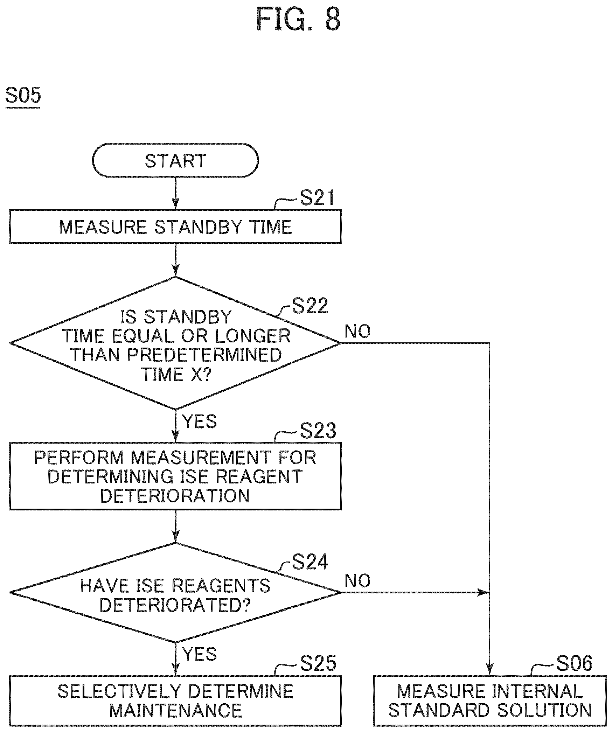

FIG. 8 illustrates a flow chart of the determination of the ISE reagent deterioration according to the first embodiment. This corresponds to processing in Step S05 of the flow chart of FIG. 7. Each step is controlled by the computer 10.

S21: The computer 10 measures a standby time of the electrolyte analysis unit 8. Specifically, the standby time is a time from the end of the initial operation (S02) to the reception of the patient sample measurement instruction (S04) or from the automatic stop (S11) to the reception of the patient sample measurement instruction (S04).

S22: When the standby time measured in Step S21 is equal to or longer than a predetermined time X (YES), the flow advances to Step S23 while, when the standby time measured in Step S21 is less than the predetermined time X (NO), the flow advances to Step S06 (measurement of the internal standard solution) so as to analyze the electrolyte of the patient sample.

S23: Measurement for determining the ISE reagent deterioration is performed.

S24: The computer 10 determines the ISE reagent deterioration on the basis of a result of the measurement for determining the ISE reagent deterioration. When determining that the ISE reagents have deteriorated (YES), the computer 10 advances to the selective determination of maintenance (S25) while, when determining that the ISE reagents have not deteriorated (NO), the computer 10 advances to Step S06 (measurement of the internal standard solution) so as to analyze the electrolyte of the patient sample. Details of Steps S23 to S25 will be described later.

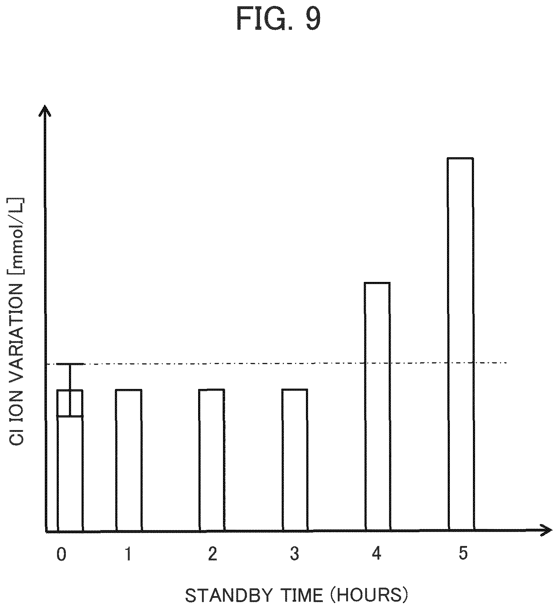

Next, with reference to FIG. 9, a description will be given of an example of a method of determining a length of the predetermined time X in Step S22. The description is given herein of such a method as illustrated in FIG. 3 to FIG. 6, in which measurement of a predetermined number of the accuracy control samples is performed at different standby times, and the predetermined time X is determined from a result of the measurement. In a graph of FIG. 9, an abscissa axis represents the standby time, while an ordinate axis represents a variation of a Cl ion measurement value. The variation of the Cl ion measurement value is assumed to be an amount of change or a rate of change (the amount of change and the rate of change will be described later) in a result of continuous measurement of the Cl ion concentration. Fluctuations of the variation of the Cl ion measurement value are also calculated. Fluctuating CI can be calculated as follows by using a standard deviation SD.

### CI=±3SD (99.7% confidence interval)

For example, in FIG. 9, the variations relative to the standby times of 1 to 3 hours have values lower than that of a variation +CI relative to the standby time of 0 hours, and therefore it can be determined that there is no deterioration. Meanwhile, the variations relative to the standby times of 4 to 5 hours have higher values than that of the variation +CI relative to the standby time of 0 hours, and therefore it can be determined that there is deterioration. On this basis, the predetermined time X can be determined to be 3 hours.

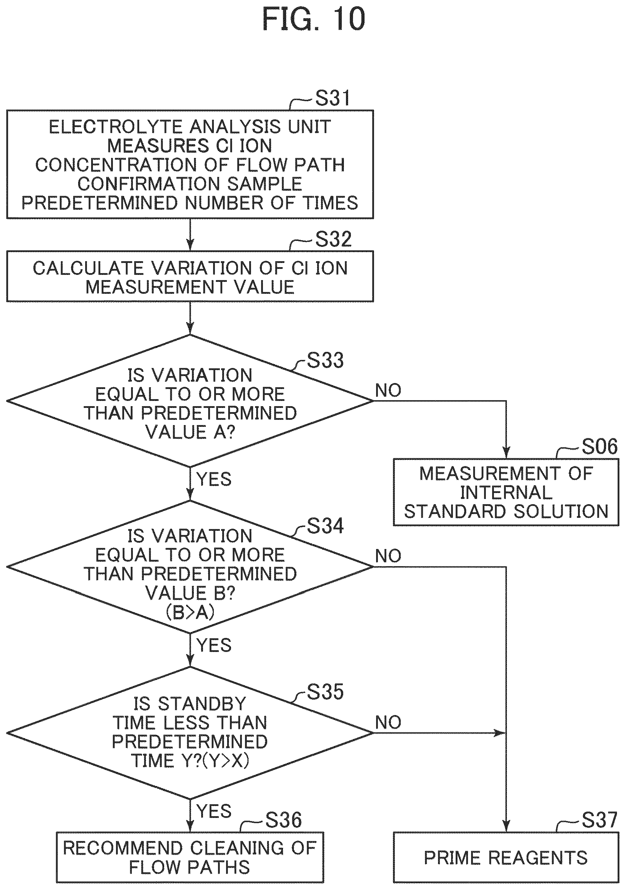

Subsequently, referring to FIG. 10, a description will be given of details of Steps S23 to S25. Step S31 corresponds to Step S23 in the flow of FIG. 8, Steps S32 to S35 correspond to Step S24 in the flow of FIG. 8, and Steps S36 to S37 correspond to Step S25 in the flow of FIG. 8.

S31: In the electrolyte analysis unit 8, the Cl ion concentration in the flow path confirmation sample is measured a predetermined number of times. The predetermined number of times needs to be at least three or more, and is determined by the user on the basis of a type of the variation to be monitored and a model of the analyzer. A type of the flow path confirmation sample is not limited as long as the sample is within a measurable concentration range determined by the analyzer. The sample to be continuously measured needs to be the same sample, but it is acceptable to continuously measure a different sample for each run.

S32: The variation of the Cl ion measurement value to be used to determine the deterioration of the ISE reagents is calculated. As the variation, the amount of change or the rate of change in measurement results of continuous measurements of the Cl ion concentration can be used.

A description will be given of the amount of change. It is assumed herein that the amount of change is a difference between a measurement value from a specified sample and a measurement value from the first sample among measurement values from the continuously measured samples. At this time, it is appropriate to specify the sample most affected by the deterioration of the ISE reagents. A position where the sample most affected by the deterioration of the ISE reagents appears differs from one analyzer model to another. For example, in a case of FIG. 6, the position where the sample most affected by the deterioration of the ISE reagents appears is in the thirteenth sample. Accordingly, when the predetermined number of times (S31) is set to 13 and the thirteenth sample is specified as the sample for which the amount of change is to be calculated, the amount of change is a difference between a measurement value (107.3 mmol/L) from the thirteenth sample and a measurement value (102.2 mmol/L) from the first sample, which is 5.1 mmol/L.

Next, a description will be given of the rate of change. It is assumed herein that the rate of change is an inclination obtained by performing primary approximation on measurement results from the measurement value from the first sample to the measurement value from the specified sample among the measurement values from the continuously measured samples. In consideration of accuracy of the approximation, it is desirable that there are three or more samples, and it is similarly appropriate to specify the sample most affected by the deterioration of the ISE reagents. For example, in a case of FIG. 6, when the rate of change is determined as an inclination obtained by performing the primary approximation on thirteen measurement results from the measurement value from the first sample to a measurement value from the thirteenth sample, the rate of change is 0.36.

S33: When the variation determined in Step S32 is equal to or more than the predetermined value A (YES), it is determined that the ISE reagents have deteriorated and that the flow paths need maintenance before the patient sample is measured, and the flow advances to Step S34. Meanwhile, when the variation determined in Step S32 is less than the predetermined value A (NO), the ISE reagents have not deteriorated, and the flow advances to the measurement of the patient sample (S06). As the predetermined value A serving as a determination threshold, any value is determined by the user on the basis of the concentration of the flow path confirmation sample and a management level of each facility.

S34: The level of the deterioration of the ISE reagents is determined. When the variation determined in Step S32 is equal to or more than a predetermined value B (YES), it is determined that the level of the deterioration of the ISE reagents is high, and the flow advances to Step S35. When the variation is less than the predetermined value B (NO), it is determined that the level of the deterioration of the ISE reagents is low, and the reagents are primed so as to replace the ISE reagents remaining in the flow paths (S37). As the predetermined value B serving as a determination threshold, any value is set by the user on the basis of the concentration of the flow path confirmation sample and the management level of each facility. However, the predetermined value B is set larger than the predetermined value A.

S35: A cause of a high deterioration level of the ISE reagents is estimated, and required maintenance is determined. As shown in the measurement results in FIG. 3 to FIG. 6, as the standby time is longer, the reagents deteriorate more rapidly, and the variation of the measurement value increases. Meanwhile, when the level of the deterioration of the ISE reagents is high despite the short standby time, it can be considered that contamination has accumulated in the ISE reagent flow paths. When the standby time is less than a predetermined time Y (YES), it is determined that the contamination has accumulated in the ISE reagent flow paths, and cleaning of the flow paths using a detergent is recommended (S36). When the standby time is equal to or longer than the time Y (NO), it is determined that the deterioration has proceeded due to the long standby time, and the reagents are primed (S37). As the predetermined time Y serving as a determination threshold, any value is set by the user on the basis of results of previous ISE deterioration determinations or operation in each facility. However, as the predetermined time Y, a value larger than that set as the predetermined time X is set.

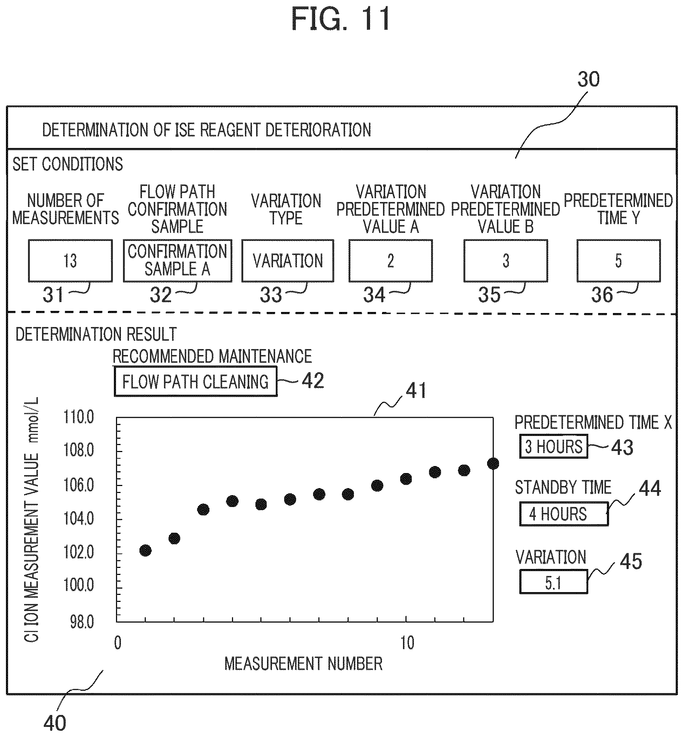

FIG. 11 illustrates an example of a screen for the ISE reagent deterioration using the electrolyte analysis unit 8. The deterioration determination screen has a condition setting area 30 and a determination result display area 40.

In the condition setting area 30, the number of measurements of the flow path confirmation sample, a name of the flow path confirmation sample, a type of the variation to be used for the deterioration determination, the predetermined value A, the predetermined value B, and the predetermined time Y are set respectively from a number-of-measurements setting unit 31, a flow-path-confirmation-sample setting unit 32, a variation type setting unit 33, a variation predetermined-value-A setting unit 34, a variation predetermined-value-B setting unit 35, and a predetermined time setting unit 36.

When the measurement of the flow path confirmation sample (S31) is performed, a result of the measurement is displayed in the determination result display area 40. A predetermined-time-X display unit 43 displays the predetermined time X applied to this determination, while a standby time display unit 44 displays the standby time in this determination. A measurement result display unit 41 displays a result of performing the measurements of the flow path confirmation sample set by the flow-path-confirmation-sample setting unit 32, the number of which is set by the number-of-measurements setting unit 31. The measurement results and the variations calculated according to the type of the variation set by the variation type setting unit 33 are displayed on a variation display unit 45, while the recommended maintenance is displayed on a recommended maintenance display unit 42.

Thus, in the first embodiment, when the standby time is equal to or longer than the predetermined time, the electrolyte analysis unit measures the flow path confirmation sample before the measurement of the patient sample, and the deterioration of the ISE reagents is determined using a result thereof. In this manner, it is possible to eliminate a measurement value abnormality due to the deterioration of the ISE reagents in the flow paths and improve reliability of the measurement results. In addition, by determining whether or not the maintenance of the ISE reagent flow paths is necessary and making a notification of maintenance items to be performed when necessary, it is possible to reduce the time required to consider the maintenance.

### Second Embodiment

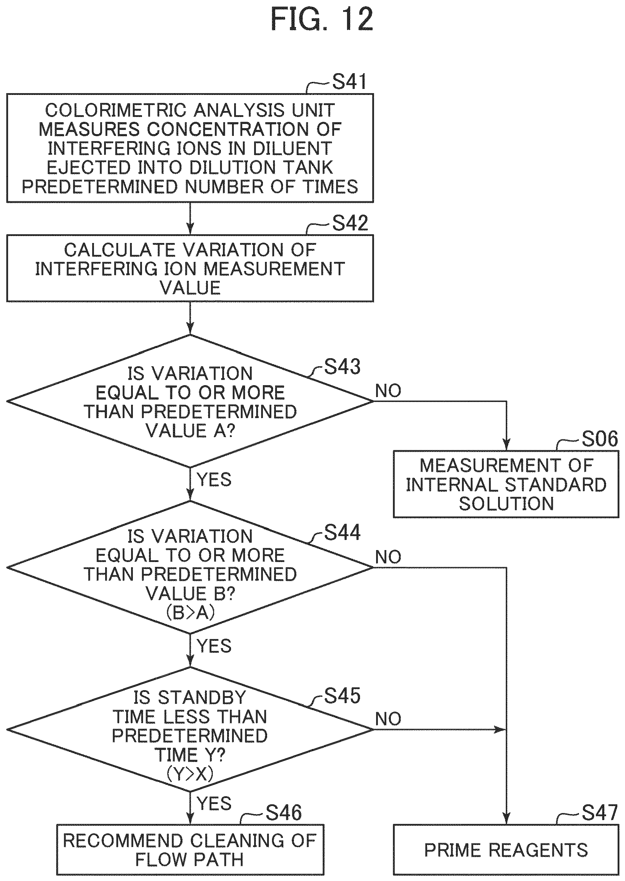

In the first embodiment, the deterioration of the ISE reagents is determined by measuring the electrolyte of the flow path confirmation sample by using the ISE reagents. In the second embodiment, the deterioration of the residual reagents is determined by the colorimetric analysis unit by measuring the concentration of the interfering ions generated with the deterioration of the ISE reagents remaining in the flow paths. A composite automatic analyzer including the colorimetric analysis unit and the electrolyte analysis unit as illustrated in FIG. 1 can implement what is disclosed in the present embodiment. The first embodiment and the second embodiment are different in the method of determining the deterioration of the residual reagents, and are otherwise the same. Accordingly, the second embodiment will mainly describe the method of determining the deterioration of the residual reagents, and omit a repetitive description. FIG. 12 is a flow chart of the determination of the deterioration of the residual reagents according to the second embodiment. Step S41 corresponds to Step S23 in the flow of FIG. 8, Steps S42 to S45 correspond to Step S24 in the flow of FIG. 8, and Steps S46 to S47 correspond to Step S25 in the flow of FIG. 8.

Note that, in the following description, the interfering ions are simply referred to as such but, in the implementation of the present flow, interfering ions resulting from the deterioration of the ISE reagents and causing a Cl ion concentration calculation error have been specified, and it is possible to detect the concentration of the interfering ions specified by colorimetric analysis.

S41: The diluent ejected into the dilution tank 18 of the electrolyte analysis unit 8 is aliquoted by the sample dispensing mechanism 7 and ejected into the reaction container 5 of the colorimetric analysis unit. From the reagent bottles placed in the reagent disks 4, the reagents are aliquoted by the reagent dispensing mechanism 6 to be ejected into the reaction container 5 in which the diluent is ejected. An absorbance changing due to a reaction between the interfering ions and the reagents in the diluent is measured with the photometer 13. From the changing of the absorbance, the concentration of the interfering ions is calculated. This measurement is continuously performed a plurality of times. The predetermined number of times needs to be at least three or more, and is determined by the user on the basis of a type of the variation to be monitored and a model of the analyzer.

S42: The variation of the concentration of the interfering ions is calculated to be used to determine the deterioration of the ISE reagents. In the same manner as in the first embodiment, as the variation, the amount of change or the rate of change in measurement results of continuous measurements of the concentration of the interfering ions can be used.

S43: When the variation determined in Step S42 is equal to or more than the predetermined value A (YES), it is determined that the ISE reagents have deteriorated and that the maintenance of the flow paths is necessary before the measurement of the patient sample, and the flow advances to Step S44. Meanwhile, when the variation determined in Step S42 is less than the predetermined value A (NO), the ISE reagents have not deteriorated, and the flow advances to the measurement of the patient sample (S06). As the predetermined value A serving as the determination threshold, any value is determined by the user on the basis of the management level of each facility.

S44: The level of the deterioration of the ISE reagents is determined. When the variation determined in Step S42 is equal to or more than the predetermined value B (YES), it is determined that the level of the deterioration of the ISE reagents is high, and the flow advances to Step S45. When the variation is less than the predetermined value B (NO), it is determined that the level of the deterioration of the ISE reagents is low, and the reagents are primed so as to replace the ISE reagents remaining in the flow paths (S47). As the predetermined value B serving as the determination threshold, any value is set by the user on the basis of the management level of each facility. However, the predetermined value B is set larger than the predetermined value A.

S45: A cause of the high deterioration level of the ISE reagents is estimated, and required maintenance is determined. When the standby time is less than the predetermined time Y (YES), it is determined that a factor which rapidly promotes the deterioration in a short time was caused in the ISE reagents flow paths, and flow path cleaning using a detergent is recommended (S46). When the standby time is equal to or longer than the time Y (NO), it is determined that the deterioration has proceeded due to the long standby time, and the reagents are primed (S47). As the predetermined time Y serving as a determination threshold, any value is set by the user on the basis of results of previous ISE deterioration determinations or operation in each facility. However, as the predetermined time Y, a value larger than that set as the predetermined time X is set.

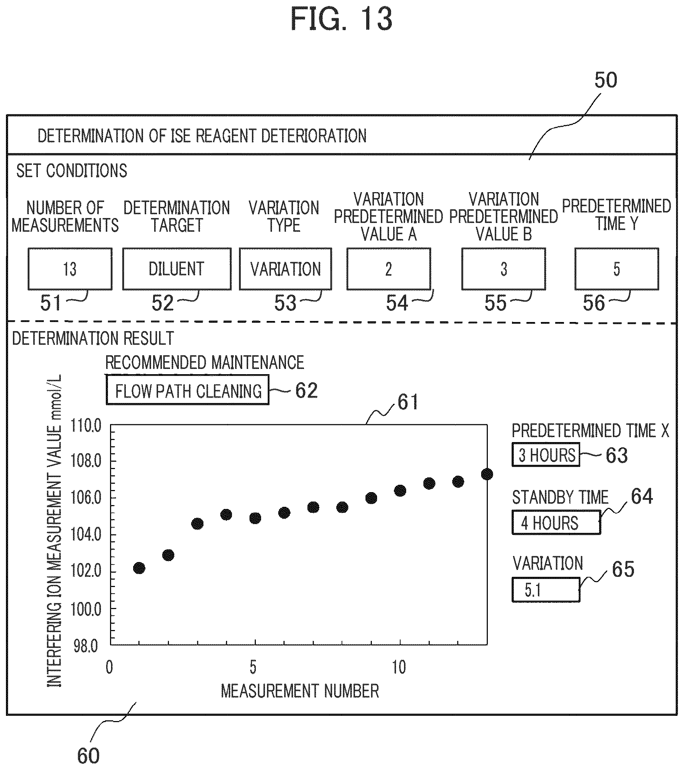

FIG. 13 illustrates an example of the screen for the determination of the deterioration of the ISE reagents using the colorimetric analysis unit. The deterioration determination screen has a condition setting area 50 and a determination result display area 60.

In the condition setting area 50, the number of measurements of the ISE reagents, names of the ISE reagents to be measured, the type of the variation to be used for the deterioration determination, the predetermined value A, the predetermined value B, and the predetermined time Y are set respectively from a number-of-measurements setting unit 51, a determination target setting unit 52, a variation type setting unit 53, a variation predetermined-value-A setting unit 54, a variation predetermined-value-B setting unit 55, and a predetermined time setting unit 56.

When the measurement of the ISE reagents (S41) is performed, a result of the measurement is displayed in the determination result display area 60. A predetermined-time-X display unit 63 displays a predetermined time X, while a standby time display unit 64 displays the standby time in this measurement. A measurement result display unit 61 displays a result of performing the measurements of the ISE reagents set by the determination target setting unit 52, the number of which is set by the number-of-measurements setting unit 51. The measurement results and the variations calculated according to the type of the variation set by the variation type setting unit 53 are displayed on a variation display unit 65, while the recommended maintenance is displayed on a recommended maintenance display unit 62.

Thus, in the second embodiment, when the standby time is equal to or longer than the predetermined time, the concentration of the interfering ions contained in the diluent is measured by the colorimetric analysis unit before the measurement of the patient sample and the deterioration of the diluent is determined using the result thereof, which can eliminate the measurement value abnormality due to the deterioration of the diluent in the flow paths and improve and reliability of the measurement results. In addition, by determining whether or not the maintenance of the ISE reagent flow paths is necessary and making a notification of maintenance items to be performed when necessary, it is possible to reduce the time required to consider the maintenance.

The second embodiment has shown an example in which the concentration of the interfering ions contained in the diluent is measured, but the same applies also to the internal standard solution. In addition, as long as the ions can be measured with the colorimetric analysis unit, types of the interfering ions are not limited.

The present invention is not limited to the above-described embodiments, and further includes various modifications. For example, the above-described embodiments and modification have been described in detail in order to facilitate the understanding of the present invention, and the present invention is not necessarily limited to those including all of the described configurations. In addition, part of the configuration of one embodiment and modification can be replaced with the configurations of other embodiments and modifications, and in addition, the configuration of the one embodiment and modification can also be added with the configurations of other embodiments and modifications. In addition, part of the configuration of each of the embodiments and modifications can be subjected to addition, deletion, and replacement with respect to other configurations.

### List of Reference Signs

- 1: Automatic analyzer
- 2: Sample disk
- 3: Sample cup
- 4: Sample disk
- 5: Reaction container
- 6: Reagent dispensing mechanism
- 7: Sample dispensing mechanism
- 8: Electrolyte analysis unit
- 9: Interface
- 10: Computer
- 11: Printer
- 12: Monitor
- 13: Photometer
- 14: Electrolyte concentration arithmetic unit
- 15: Potentiometer
- 16: Internal standard solution
- 17: Diluent
- 18: Dilution tank
- 19: Cl ion electrode
- 20: K ion electrode
- 21: Na ion electrode
- 22: Pinch valve
- 23: Reference electrode
- 24: Reference electrode solution
- 25: Deaerator tank
- 26: Sipper syringe
- 30, 50: Condition setting area
- 31, 51: Number-of-measurements setting unit
- 32: Flow-path-confirmation-sample setting unit
- 33, 53: Variation type setting unit
- 34, 54: Variation predetermined-value-A setting unit
- 35, 55: Variation predetermined-value-B setting unit
- 36, 56: Predetermined time setting unit
- 40, 60: Determination result display area
- 41, 61: Measurement result display unit
- 42, 62: Recommended maintenance display unit
- 43, 63: Predetermined-time-X display unit
- 44, 64: Standby time display unit
- 45, 65: Variation display unit
- 52: Determination target setting unit

## Claims

1. An automatic analyzer comprising:
an electrolyte analysis unit that measures an electrolyte concentration by an ion selective electrode method and can measure at least a chloride ion concentration; and
a control unit,
wherein, on receipt of an instruction to measure a patient sample that requires measurement using the electrolyte analysis unit, the control unit determines, when a standby time of the electrolyte analysis unit is equal to or longer than a predetermined time, deterioration of a residual reagent remaining in a flow path of the electrolyte analysis unit prior to the measurement of the patient sample.

2. The automatic analyzer according to claim 1,
wherein the electrolyte analysis unit includes a dilution tank, an ion selective electrode, a reference electrode, a flow path that ejects an internal standard solution into the dilution tank, a flow path that ejects a diluent into the dilution tank, a flow path that aspirates a liquid in the dilution tank to the ion selective electrode, and a flow path that aspirates a reference electrode solution to the reference electrode.

3. The automatic analyzer according to claim 1,
wherein the control unit causes the electrolyte analysis unit to continuously perform the measurement of the chloride ion concentration of a flow path confirmation sample and determines the deterioration of the residual reagent on the basis of a result of the measurement of the chloride ion concentration of the flow path confirmation sample.

4. The automatic analyzer according to claim 3,
wherein the control unit measures the patient sample when a variation of the continuously measured chloride ion concentration of the flow path confirmation sample is less than a predetermined value or recommends replacement of the residual reagent remaining in the flow path or maintenance of the flow path when the variation of the chloride ion concentration of the flow path confirmation sample is equal to or more than the predetermined value.

5. The automatic analyzer according to claim 4,
wherein the control unit calculates, from a measurement value resulting from the continuous measurement of the flow path confirmation sample, the variation as an inclination obtained by performing first-order approximation on a difference between a measurement value resulting from a first measurement and a measurement value resulting from a measurement corresponding to a specified number of times or on the measurement value resulting from the first measurement and measurement values resulting from measurements up to the measurement corresponding to the specified number of times.

6. The automatic analyzer according to claim 1, further comprising:
a colorimetric analysis unit capable of measuring a concentration of interfering ions that cause a chloride ion concentration measurement error in the electrolyte analysis unit,
wherein the control unit causes the colorimetric analysis unit to continuously measure the interfering ion concentration of the residual reagent a predetermined number of times, and determines the deterioration of the residual reagent on the basis of a result of the measurement of the interfering ion concentration of the residual reagent.

7. The automatic analyzer according to claim 6,
wherein the control unit measures the patient sample when a variation of the continuously measured interfering ion concentration of the residual reagent is less than a predetermined value or recommends replacement of the residual reagent remaining in the flow path or maintenance of the flow path when the variation of the interfering ion concentration of the residual reagent is equal to or more than the predetermined value.

8. The automatic analyzer according to claim 7,
wherein the control unit calculates, from a measurement value resulting from the continuous measurement of the residual reagent, the variation as an inclination obtained by performing first-order approximation on a difference between a measurement value resulting from a first measurement and a measurement value resulting from a measurement corresponding to a specified number of times or on the measurement value resulting from the first measurement and measurement values resulting from measurements up to the measurement corresponding to the specified number of times.

9. A measuring method for a patient sample using an automatic analyzer including an electrolyte analysis unit that measures an electrolyte concentration by an ion selective electrode method and can measure at least a chloride ion concentration, and a control unit,
the measuring method comprising:
causing the control unit to measure, on receipt of an instruction to measure a patient sample that requires measurement using the electrolyte analysis unit, a standby time of the electrolyte analysis unit; and
causing the control unit to determine deterioration of a residual reagent remaining in a flow path of the electrolyte analysis unit prior to the measurement of the patient sample when the standby time of the electrolyte analysis unit is equal to or longer than a predetermined time.

10. The measuring method according to claim 9,
wherein the electrolyte analysis unit continuously performs the measurement of the chloride ion concentration of a flow path confirmation sample, and
wherein the control unit determines the deterioration of the residual reagent on the basis of a result of the measurement of the chloride ion concentration of the flow path confirmation sample.

11. The measuring method according to claim 10,
wherein the control unit measures the patient sample when a variation of the continuously measured chloride ion concentration of the flow path confirmation sample is less than a predetermined value or recommends replacement of the residual reagent remaining in the flow path or maintenance of the flow path when the variation of the chloride ion concentration of the flow path confirmation sample is equal to or more than the predetermined value.

12. The measuring method according to claim 11,
wherein the control unit calculates, from a measurement value resulting from the continuous measurement of the flow path confirmation sample, the variation as an inclination obtained by performing first-order approximation on a difference between a measurement value resulting from a first measurement and a measurement value resulting from a measurement corresponding to a specified number of times or on the measurement value resulting from the first measurement and measurement values resulting from measurements up to the measurement corresponding to the specified number of times.

13. The measuring method according to claim 9,
wherein the automatic analyzer further includes:
a colorimetric analysis unit capable of measuring a concentration of interfering ions that cause a chloride ion concentration measurement error in the electrolyte analysis unit,
wherein the colorimetric analysis unit continuously measures the interfering ion concentration of the residual reagent a predetermined number of times, and
wherein the control unit determines the deterioration of the residual reagent on the basis of a result of the measurement of the interfering ion concentration of the residual reagent.

14. The measuring method according to claim 13,
wherein the control unit measures the patient sample when a variation of the continuously measured interfering ion concentration of the residual reagent is less than a predetermined value or recommends replacement of the residual reagent remaining in the flow path or maintenance of the flow path when the variation of the interfering ion concentration of the residual reagent is equal to or more than the predetermined value.

15. The measuring method according to claim 14,
wherein the control unit calculates, from a measurement value resulting from the continuous measurement of the residual reagent, the variation as an inclination obtained by performing first-order approximation on a difference between a measurement value resulting from a first measurement and a measurement value resulting from a measurement corresponding to a specified number of times or on the measurement value resulting from the first measurement and measurement values resulting from measurements up to the measurement corresponding to the specified number of times.
